(19) Europäisches Patentamt
European Patent Office
Office européen des brevets

(11) Publication number: **0 423 943 A2**

(12) # EUROPEAN PATENT APPLICATION

(21) Application number: **90310248.1**

(22) Date of filing: **19.09.90**

(51) Int. Cl.[5]: **A61K 31/405**, A61K 31/165, A61K 31/66

The title of the invention has been amended (Guidelines for Examination in the EPO, A-III, 7.3).

(30) Priority: **21.09.89 GB 8921326**

(43) Date of publication of application: **24.04.91 Bulletin 91/17**

(84) Designated Contracting States: **CH DE FR GB IT LI NL**

(71) Applicant: **Beecham Group p.l.c.**
**SB House Great West Road**
**Brentford Middlesex TW8 9BD(GB)**

(72) Inventor: **Harper, Gregory Philip SmithKline Beecham Pharmac.**
**Coldharour Road, The Pinnacles**
**Harlow, Essex CM19 5AD(GB)**

(74) Representative: **Russell, Brian John et al**
**SmithKline Beecham, Corporate Patents,**
**Great Burgh, Yew Tree Bottom Road**
**Epsom Surrey KT18 5XQ(GB)**

(54) **Use of collagenase inhibitors in the treatment of demyelinating diseases, in particular multiple sclerosis.**

(57) The use of a compound having activity as an inhibitor of a member of the mammalian collagenase family of enzymes, including collagenase, in the treatment of demyelinating diseases of the nervous system, in particular multiple schlerosis and related demyelinating disorders.

EP 0 423 943 A2

## NOVEL TREATMENT

The present invention relates to the use of inhibitors of the collagenase family of enzymes for treating multiple sclerosis and other disorders involving myelin degradation.

Multiple sclerosis is a chronic disease of the central nervous system in which the myelin sheaths surrounding axonal processes are degraded within discrete plaques. The clinical manifestations of the disease follow from this underlying pathological process. Similar mechanisms may underlie related diseases, such as optic neuritis, neuromyelitis optica (Devic's disease), diffuse and transitional sclerosis (Schilder's disease) and acute disseminated encephalomyelitis. Demyelinating peripheral neuropathies include acute inflammatory demyelinating polyradiculoneuropathies (Landry-Guillain-Barre-Strohl syndrome for motor defects, and analogous syndromes for sensory and autonomic (pandysautonomia) deficits), and the chronic and recurrent inflammatory demyelinating polyradiculoneuropathies. Demyelinating diseases can be divided into those involving primary segmental demyelination - in which the myelin destruction is probably causative - and those involving secondary segmental demyelination, in which myelin loss is a consequence of axonal atrophy, e.g. in Friedrich's ataxia.

The mammalian collagenase family of enzymes comprises a number of proteases, exemplified by interstitial (type I) collagenase itself, the stromelysins (also known as proteoglycanases or transins), fibroblast and polymorphonuclear leucocyte gelatinases (also known as collagen-IV-ases), and 'pump-1' (putative metalloprotease 1, uterine metalloprotease) [Goldberg et al , J. Biol. Chem. 2610 , 6600, 1986; Whitham et al , Biochem. J. 240 , 913, 1986; Breathnach et al, Nucleic Acids Res., 15 , 1139, 1987; Muller et al , Biochem. J., 253 , 187, 1988; Collier et al , J. Biol. Chem. 263 , 6579, 1988; Murphy et al , Biochem. J., 258 , 463, 1989; Quantin et al , Biochem. (N.Y.), 28 , 5327, 1989; Birkedal-Hansen, J. Oral Pathol., 17 , 445, 1988]. Membership of the mammalian collagenase family of proteases is evident by possession of a number of highly characteristic and experimentally verifiable properties, which can be adopted as criteria for allocation to this enzyme family, selected from:

(a) Optimal proteolytic activity around neutral pH.

(b) Dependence of the enzyme's activity on the presence of zinc, as evident by the loss of activity on treatment with divalent metal ion chelators, such as 1,10-phenanthroline (preferential chelation of zinc), or EDTA (less restricted chelating properties; EDTA and EGTA also inactivate the enzymes via chelation of calcium ions required for enzyme stability).

(c) Inhibition by TIMP (Tissue Inhibitor of Metalloproteases), a proteinaceous inhibitor thought to play a significant role in the physiological control of the collagenase family of enzymes. Other families of metalloproteases are not inhibited by TIMP, at least as far as such studies have been performed.

(d) Absence of significant inhibition by known inhibitors of other families of neutral, zinc-containing metalloproteases, such as thermolysin, angiotensin-converting enzyme and 'enkephalinase' (EC 3.4.24.11). One of the inhibitors most often used is phosphoramidon which inhibits thermolysin and enkephalinase.

(e) Biosynthesis and secretion as latent precursor forms (zymogens), requiring extracellular activation. Activation has been achieved by a number of endoproteases, organomercurials and chaotropic agents.

Recently, a metalloprotease acting at neutral pH has been identified as a component of myelin membranes, which is able to degrade myelin basic protein, a major component of the myelin sheath (Chantry A. et al ., J. Neurochem. 50 , 688-694, 1988). The enzyme is inhibited by 1,10-phenanthroline, but not by phosphoramidon. This enzyme may therefore play a significant role in the pathogenesis of the demyelinating diseases.

United States Patent no. 4,263,293 (Squibb) describes compounds of formula (I):

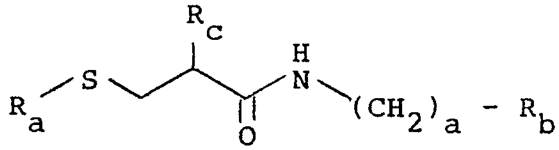

(I)

wherein $R_a$ is hydrogen, $C_{2-10}$ alkanoyl or arylcarbonyl; $R_b$ is 1-pyrrolidinyl, 1-piperidinyl, 4-morpholinyl, 1-piperazinyl or 4-alkyl-1-piperazinyl; $R_c$ is $C_{3-8}$ alkyl, $C_{3-7}$ cycloalkyl, aryl or aralkyl; and a is an integer of 1

to 20.

Compounds of formula (I) are described as effective in inhibiting mammalian collagenase and for use in reducing the adverse effects of collagenase in a mammal host.

United States Patent No. 4,595,700 (Searle) describes compounds of formula (II):

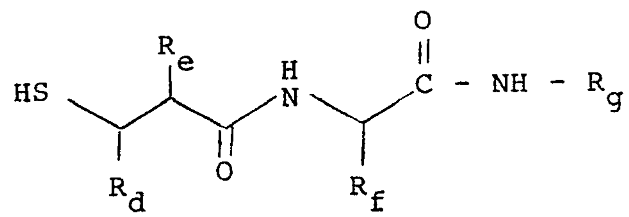

(II)

wherein $R_d$ represents lower alkyl, phenyl or phenyl lower alkyl; $R_e$ and $R_g$ represent lower alkyl; and $R_f$ represents lower alkyl, benzyloxy $C_{1-6}$ alkyl, $C_{1-6}$ alkoxybenzyl or benzyloxybenzyl.

Compounds of formula (II) are described as collagenase inhibitors having utility in the treatment of rheumatoid arthritis and related diseases in which collagenolytic activity is a contributing factor.

International Patent Application WO88/06890 (Research Corp. Technologies Inc) describes compounds of formula (III):

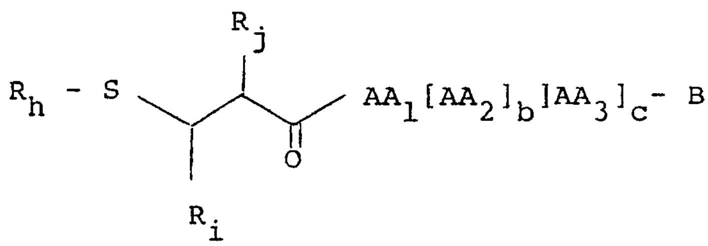

(III)

wherein $R_h$ is hydrogen, $C_{1-10}$ alkyl, $C_{2-10}$ alkanoyl or $C_{7-11}$ aroyl; $R_i$ is hydrogen or $C_{1-6}$ alkyl; $R_j$ is hydrogen, $C_{2-10}$ alkyl, $C_{3-6}$ cycloalkyl, aryl or arylalkyl, wherein the aryl moiety has from 6 to 10 carbon atoms; $AA_1$ is a hydrophobic amino acid; $AA_2$ is an amino acid selected from alanine, glycine, leucine, isoleucine and phenylalanine; $AA_3$ is any amino acid; B is $-NH_2$, -OH, $-OCH_3$ or $-OCH_2CH_3$; b is 0 or 1; and c is 0, 1 or 2.

Compounds of formula (III) are described as effective inhibitors of mammalian collagenase activity which may be employed in the treatment of any mammalian disease in which collagenase has been implicated as a causative factor.

European Patent Publication No. 273689 (Beecham) describes compounds of formula (IV):

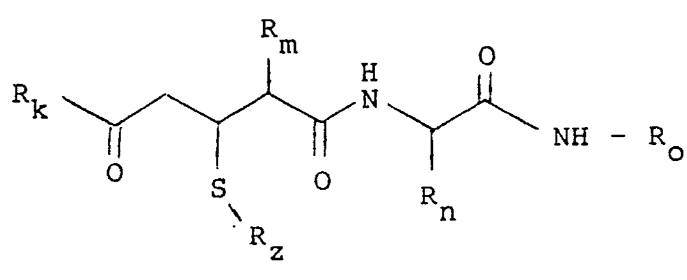

(IV)

wherein $R_k$ is hydroxy, alkoxy, aryloxy, aralkyloxy, $-NR_pR_q$ where $R_p$ and $R_q$ are hydrogen or alkyl, or $R_p$ and $R_q$ together with the nitrogen atom to which they are bonded form a 5- to 7-membered ring optionally including a further heteroatom, $-NHCH(R_r)COR_s$ where $R_r$ is hydrogen, alkyl optionally substituted by hydroxy, alkoxy, $-NR_pR_q$ guanidine, $-CO_2H$, $-CONH_2$, -SH, -S-alkyl or $-CH_2-A_r$ where $A_r$ is optionally substituted aryl, and $R_s$ is hydroxy, alkoxy or $-NR_pR_q$; $R_z$ is hydrogen, alkanoyl or aroyl; $R_m$ is $C_{3-6}$ alkyl; $R_n$ is hydrogen, alkyl, $-CH_2R_t$ where $R_t$ is optionally substituted phenyl or heteroaryl, or $R_n$ is a group -CH-$(R_u)$O-$R_v$ where $R_u$ is hydrogen or alkyl and $R_v$ is hydrogen, alkyl or $-CH_2Ph$ where Ph is optionally

substituted phenyl; $R_o$ is hydrogen, alkyl, or $-CH(R_w)COR_x$ where $R_w$ is hydrogen or alkyl and $R_x$ is hydroxy, alkoxy or $-NR_pR_q$.

European Patent Publication No. 322184 (Beecham) describes compounds of formula (IV) wherein $R_k$ is hydrogen, alkyl or optionally substituted aryl and variables $R_l$, $R_m$, $R_n$ and $R_o$ are as hereinbefore defined for compounds of formula (IV) in European Patent publication No. 273689.

Compounds of formula (IV) are described as collagenase inhibitors having utility in the treatment of disorders resulting from collagenolytic activity.

European Patent Publication No. 358305 (corresponding to United States Patent Application No. 367961) (Beecham) describes compounds of formula (V):

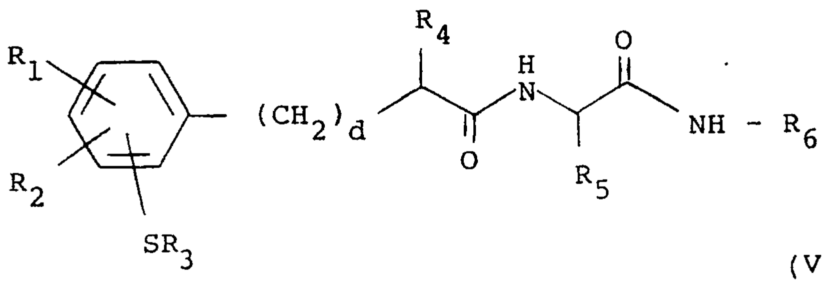

(V)

Wherein $R_1$ and $R_2$ are independently hydrogen, alkyl, alkoxy, halogen or $CF_3$; $R_3$ is hydrogen, alkanoyl, aroyl in which the aryl moiety is optionally substituted, or a group R-S- where R is an organic residue such that the group R-S- provides an in vivo -cleavable disulphide bond; $R_4$ is $C_{3-6}$ alkyl, $R_5$ is hydrogen, alkyl, $-CH_2R_7$ where $R_7$ is optionally substituted phenyl or heteroaryl, or $R_7$ is a group $-CH(R_8)OR_9$ where $R_8$ is hydrogen or alkyl, and $R_9$ is hydrogen, alkyl, or $-CH_2Ph$ there Ph is optionally substituted phenyl; $R_6$ is hydrogen, alkyl, $-CH(R_{10})COR_{11}$ where $R_{10}$ is hydrogen or alkyl and $R_{11}$ is hydroxy, alkoxy, or $-NR_{12}R_{13}$ where each of $R_{12}$ and $R_{13}$ is hydrogen or alkyl, or $R_{12}$ and $R_{13}$ together with the nitrogen atom to which they are bonded form a 5-,6- or 7-membered ring with an optional oxygen, sulphur or optionally substituted nitrogen atom in the ring; or $R_5$ and $R_6$ are joined as $-(CH_2)_e-$ where e is an integer from 4 to 12; and d is an integer from 1 to 3.

Compounds of formula (V) are described as collagenase inhibitors having utility in the treatment of disorders resulting from collagenolytic activity.

United States Patent No. 4,599,361 (Searle) and European Patent Publication No. 214639 (Searle) describe compounds of formula (VI):

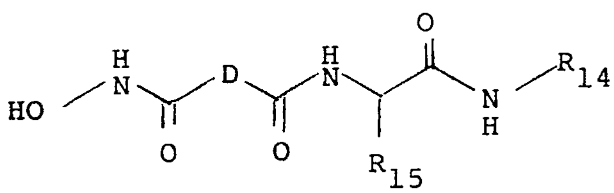

(VI)

wherein $R_{14}$ is $C_{1-6}$ alkyl; $R_{15}$ is $C_{1-6}$ alkyl, benzyl, benzyloxybenzyl, ($C_{1-6}$ alkoxy)benzyl or benzyloxy ($C_{1-6}$ alkyl); and D is a group $-(CHR_{16}-CHR_{17})-$ or $-(CR_{16}-CR_{17})-$ where $R_{16}$ is hydrogen, $C_{1-6}$ alkyl, phenyl or phenyl($C_{1-6}$ akyl) and $R_{17}$ is hydrogen, ($C_{1-6}$ alkyl), phenyl ($C_{1-6}$ alkyl), cycloalkyl or cycloalkyl ($C_{1-6}$ alkyl).

Compounds of formula (VI) are described as inhibitors of mammalian collagenase having utility in the treatment of rheumatoid arthritis and related diseases in which collagenolytic activity is a contributing factor.

European Patent Publication No. 274453 (Laboratoire Roger Bellon) describes compounds of formula (VII):

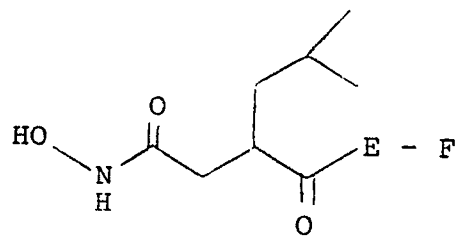

(VII)

Wherein E represents an amino acid residue selected from valine, lysine, norleucine or methionine and F represents amino or $C_{1-2}$ alkylamino in which the alkyl moiety is substituted by phenyl or trifluoromethylphenyl.

Compounds of formula (VII) are described as inhibitors of colleganase having utility in the treatment of disorders implicated in the destruction of collagen by collagenase.

European Patent Publication No. 231081 (ICI Americas Inc.) describes compounds of formula (VIII):

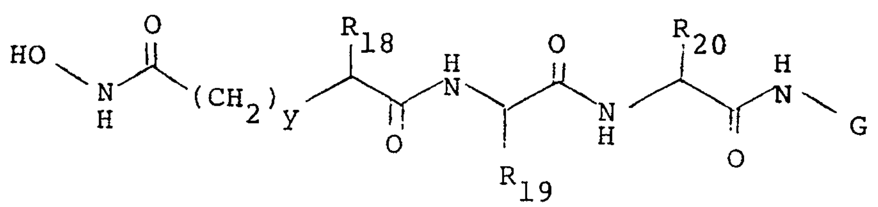

(VIII)

wherein $R_{18}$ is a straight or branched chain $C_{2-7}$ alkyl group; $R_{19}$ and $R_{20}$ are each an amino acid residue; y is 1 or 2; and G is hydrogen or $-CH(R_{21})CONH_2$ where $R_{21}$ is an amino acid residue.

Compounds of formula (VIII) are described as inhibitors of the activity of metalloproteases, including collagenase, having utility in the treatment of diseases in which the activity of such enzymes has been implicated.

European Patent Publication No. 236872 (Hoffman-La Roche) describes compounds of formula (IX):

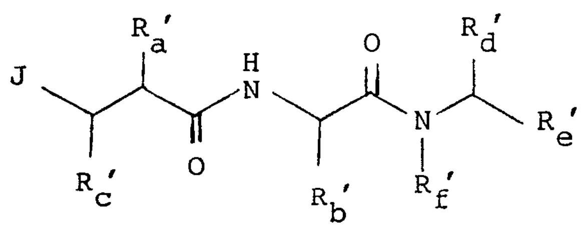

(IX)

wherein $R_a'$ is $C_{2-5}$ alkyl; $R_b'$ is a side-chain of a natural α-amino acid in which any functional group is optionally protected, any amino group is optionally acylated and any carboxyl group is optionally amidated with the proviso that $R_b'$ is not hydrogen or methyl; $R_c'$ is hydrogen, amino, hydroxy, mercapto, $C_{1-6}$ alkyl, $C_{1-6}$ alkoxy, $C_{1-6}$ alkylamino-, $C_{1-6}$ alkylthio-, aryl $C_{-16}$ alkyl-, amino $C_{1-6}$ alkyl-, hydroxy $C_{1-6}$ alkyl-, mercapto $C_{1-6}$ alkyl- or carboxy $C_{1-6}$ alkyl- wherein any amino, hydroxy, mercapto or carboxy group is optionally protected, any amino group is optionally acylated and any carboxy group is optionally amidated; $R_d'$ is hydrogen or methyl; $R_e'$ is hydrogen, $C_{1-6}$ akyl, $C_{1-6}$ alkoxy $C_{1-6}$ alkyl, di-($C_{1-6}$ alkoxy)methyl, carboxy, $C_{2-7}$ alkanoyl, $C_{1-6}$ alkoxycarbonyl, arylmethoxycarbonyl, $C_{1-6}$ alkylaminocarbonyl or arylaminocarbonyl; $R_f'$ is hydrogen or methyl; or $R_b'$ and $R_d'$ together are $-(CH_2)_f-$ where f is an integer from 4 to 11; or $R_d'$ and $R_e'$ together are $(CH_2)_3$; and J is -C(O)NHOH or -N(OH)CHO.

Compounds of formula (IX) are described as collagenase inhibitors having utility in the treatment of degenerative joint diseases such as rheumatoid arthritis and osteoarthritis.

European Patent Publication No. 126974 (Searle) describes compounds of formula (X):

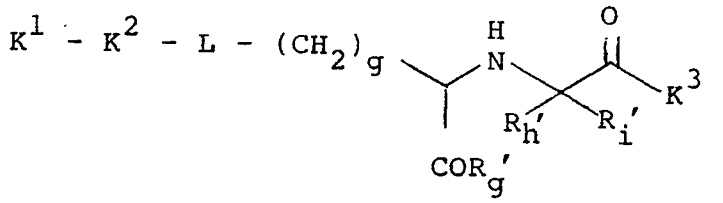

(X)

wherein g is an integer from 1 to 4; $R_g'$ represents hydroxy, alkoxy, aralkyloxy or hydroxyamino; $R_h'$ represents hydrogen or alkyl; $R_i'$ represents hydrogen, alkyl optionally substituted by one or more groups selected from hydroxy, alkoxy, aryloxy, aralkyloxy, mercapto, alkylthio, arylthio, alkylsulphinyl, alkylsulphonyl, carboxy, carboxamido, carboxyalkyl, carboxyaralkyl, aralkoxycarbonylamino, amino, dialkylamino, acylamino, aroylamino and trihalomethyl. aralkyl optionally substituted on the aryl moiety by one or more groups selected from halogen, alkyl hydroxy, alkoxy, aralkoxy, amino, aminomethyl, cyano, alkylamino, dialkylamino, carboxy, sulphonamido, alkylthio, nitro and phenyl, or heteroaralkyl; L represents $NR_j'$ wherein $R_j'$ represents hydrogen or alkyl, or L represents a direct chemical bond; when L represents $NR_j'$, $K^1$ represents a group $R_k'$ wherein $R_k'$ is hydrogen, alkyl, aralkyl, acyl, aroyl, aralkylacyl, alkoxycarbonyl, aralkoxycarbonyl, aryl optionally substituted by one or more groups selected from halogen, alkyl, hydroxy, alkoxy, aralkoxy, aralkoxyamino, aminomethyl, cyano, acylamino, dialkylamino, carboxy, sulphonamido, alkylthio, nitro and phenyl; or alternatively $K^1$ represents a group $R_z'R_m'N\text{-}C(R_n')(R_o')\text{-}C(O)\text{-}$ wherein $R_z'$ is $R_k'$ as defined above, $R_m'$ and $R_n'$ independently represent hydrogen, alkyl or aralkyl or together represent an alkylene chain of 2 to 4 carbon atoms so as to form with the adjacent nitrogen a 4- to 6- membered ring, and $R_o'$ is $R_i'$ as defined above; $K_2$ represents $N(R_p')\text{-}C(R_q')(Rr')\text{-}C(O)\text{-}$ wherein $R_p'$ and $R_q'$ independently represent those groups as defined for $R_m'$ above, or $R_p'$ and $R_q'$ together represent an alkylene chain of 2 to 4 carbon atoms so as to form with the adjacent nitrogen a 4- to 6- membered ring, and $R_r'$ is $R_o'$ as defined above; or alternatively $K^1$ and $K^2$ together represent hydrogen, alkyl, aralkyl, heteroaralkyl, alkylsulphonyl, arylsulphonyl, aralkylsulphonyl, or a group $R_s'CO\text{-}$ wherein $R_s'$ represents hydrogen, alkyl, aralkyl, aryl, alkoxy, aralkoxy, alkylamino, arylamino, aralkylamino, phenethenyl, phenethynyl, dialkylamino, substituted aryl as defined above for $R_k'$, substituted aralkyl as defined above for $R_i'$, or substituted aralkoxy wherein the substituent on the aromatic moiety is as for substituted aryl under $R_k'$ above; or alternatively when L represents a direct chemical bond, $K^1$ and $K^2$ together represent hydrogen, alkyl, aryl, alkoxy, aralkoxy, substituted aryl or substituted aralkoxy wherein the substituents on the aromatic moieties are as defined for substituted aralkyl under $R_i'$, hydroxy, mercapto, alkylthio, arylthio, aralkylthio, carboxy or carboxyalkyl; and $K^3$ represents $R_t'$ or a group $\text{-}N(R_p')\text{-}C(R_q')(R_r')\text{-}C(O)\text{-}R_u'$ wherein $R_t'$ represents amino, alkylamino, dialkylamino, hydroxyamino or aralkylamino, $R_p'$, $R_q'$ and $R_r'$ are as defined above under $K^2$, and $R_u'$ represents amino, alkylamino, dialkylamino, alkylamino optionally substituted by amino, hydroxy, alkoxy, carboxy, carboxamido, carboxyalkyl, alkylthio, alkylsulphinyl or alkylsulphonyl, hydroxyamino, alkoxyamino, aralkylamino, alkoxy, aralkoxy or alkylaminoalkoxy; with the proviso that when $K^3$ is alkylamino, then one of $R_h'$ and $R_i'$ is not hydrogen when the other is alkyl or hydroxyalkyl.

Compounds of formula (X) are described as inhibitors of mammalian collagenase which are useful in the treatment of rheumatoid arthritis and associated diseases.

European Patent Publication No. 159396 (Searle) describes compounds of formula (XI):

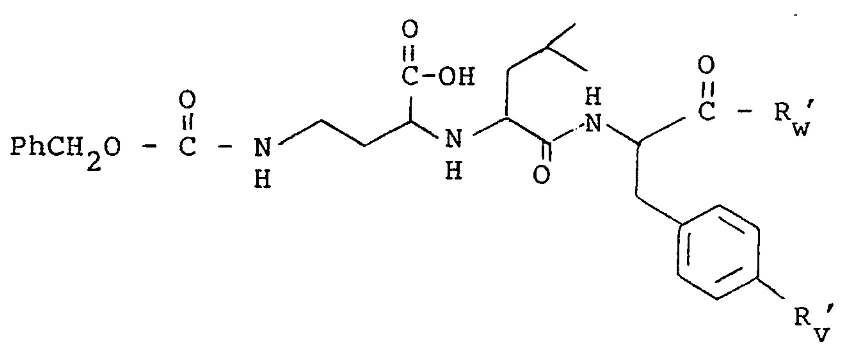

(XI)

wherein $R_v'$ represents hydroxy, alkoxy, cycloalkoxy, aralkoxy or alkoxy substituted by alkylaminocarbonyl or a group -C(O)-NH-CH)(alkyl)-C(O)-NH-alkyl; and $R_w'$ represents alkylamino or aralkylamino.

Compounds of formula (XI) wherein Rv' is hydroxy, alkoxy or aralkoxy fall within the scope of

compounds of formula (X) as defined above.

Compounds of formula (XI) are described as inhibitors of mammalian collagenase which are useful in the treatment of rheumatoid arthritis and related diseases.

European Patent Publication No. 232027 (ICI Americas Inc) describes compounds of formula (XII):

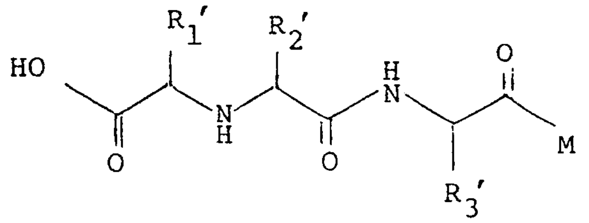

(XII)

wherein $R_1'$ is $C_{4-6}$ alkyl; $R_2'$ is $C_{4-6}$ alkyl; $R_3'$ is $C_{1-6}$ alkyl or $C_{6-10}$ aryl optionally substituted by $C_{1-4}$ alkyl or $C_{1-4}$ heteroalkyl having one or more heteroatoms independently selected from oxygen, sulphur and nitrogen; and M is selected from $-NHR_4'$, $-OCH_2R_4'$, $-NH-CH(R_5')-C(O)-NHR_4'$, $-NH-CH(R_5')-C(O)-NH-CH-(R_6')-C(O)-NHR_4'$, $-NH-CH(R_5')-C(O)-OCH_2R_4'$, and $-NH-CH(R_5')-C(O)-NHCH(R_6')-C(O)-OCH_2R_4'$, where $R_4'$ is hydrogen, $C_{1-10}$ alkyl or $C_{6-10}$ aryl optionally substituted by $C_{1-4}$ alkyl or $C_{1-4}$ heteroalkyl having one or more heteroatoms independently selected from oxygen, sulphur and nitrogen; and $R_5'$ and $R_6'$ are independently $C_{1-4}$ alkyl or $C_{6-10}$ aryl optionally substituted by $C_{1-4}$ alkyl or $C_{1-4}$ heteroalkyl having one or more heteroatoms independently selected from oxygen, sulphur and nitrogen, subject to the proviso that if the compound of formula (XII) is a tripeptide terminating in $-NHR_4'$, then $R_5'$ may not be isobutyl, and if the compound of formula (XII) is a tetrapeptide terminating in $-NHR_4'$, then $R_6'$ may not be isobutyl. compounds of formula (XII) are described as selective, inhibitors of proteogylcanase, that are not inhibitors of collagenase which are useful whenever it is desired to inhibit the activity of proteoglycanase and particularly when it is desired to selectively inhibit the activity of proteoglycanase in the presence of collagenase.

European Patent Publication No. 320118 (Beecham) describes compounds of formula (XIII):

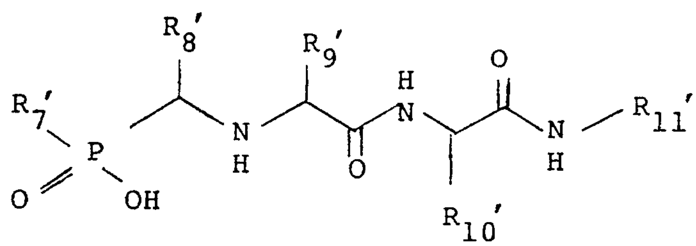

(XIII)

wherein $R_7'$ is hydrogen or hydroxy; $R_8'$ is hydrogen or alkyl; $R_9'$ is $C_{3-6}$ alkyl; $R_{10}'$ is hydrogen, alkyl, $-CH_2-Q$ where Q is optionally substituted phenyl or heteroaryl, or $R_{10}'$ is a group $-CH(R_{12}')-OR_{13}'$ where $R_{12}'$ is hydrogen or alkyl and $R_{13}'$ is hydrogen, alkyl or $-CH_2-Ph$ where Ph represents optionally substituted phenyl; and $R_{11}'$ is hydrogen or alkyl.

Compounds of formula (XII) are described as collagenase inhibitors having utility in the treatment of disorders resulting from collagenolytic activity.

European Patent Publication No. 276436 (Hoffmann-La Roche) describes compounds of formula (XIV):

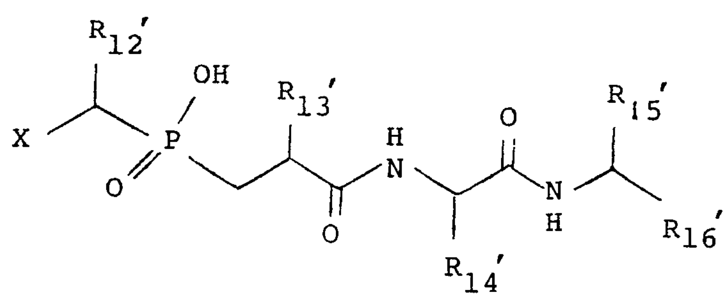

(XIV)

wherein $R_{12}'$ represents hydrogen, $C_{1-6}$ alkyl, or aryl $C_{1-6}$ alkyl; $R_{13}'$ represents $C_{2-5}$ alkyl; $R_{14}'$ represents the side-chain of a natural α-amino acid in which any functional group present is optionally protected, any amino group present is optionally acylated or sulphonylated, or any carboxyl group present is optionally amidated, subject to the proviso that $R_{14}'$ does not represent hydrogen or methyl; $R_{15}'$ represents hydrogen or methyl; or $R_{14}'$ and $R_{15}'$ together represent $-(CH_2)_n-$ where n is an integer from 4 to 11; $R_{16}'$ represents hydrogen, $C_{1-6}$ alkyl, carboxy, $C_{1-6}$ alkoxycarbonyl or $C_{1-6}$ alkylaminocarbonyl; and X represents either a cyclic imido group derived from an aliphatic or aromatic dicarboxylic acid, from an N-carboxyamino acid, from an azadicarboxylic acid or from an O-carboxyhydroxy acid, or a group $R_{21}'-NR_{20}'-CH(R_{19}')-C(O)-NR_{18}'-CH(R_{17}')-C(O)-NH-$ in which $R_{17}'$ represents the side-chain of a natural α-amino acid in which any functional group present is optionally protected, any amino group present is optionally acylated or sulphonylated, or any carboxyl group present is optionally amidated; $R_{18}'$ represents hydrogen, or $R_{17}'$ and $R_{18}'$ together represent a trimethylene group; $R_{19}'$ represents the side-chain of a natural α-amino acid in which any functional group present is optionally protected, any amino group present is optionally acylated or sulphonylated, or any carboxyl group present is optionally amidated; $R_{20}'$ represents hydrogen; or $R_{19}'$ and $R_{20}'$ together represent a trimethylene group; and $R_{21}'$ represents a protecting group or an acyl, $C_{1-6}$ alkylsulphonyl or arylsulphonyl group, with L-stereochemistry at the chiral centres bearing $R_{17}'$ and $R_{19}'$.

Compounds of formula (XIV) are described as inhibitors of the enzyme collagenase having utility in the control or prevention of degenerative joint diseases such as rheumatoid arthritis and osteoarthritis.

Further patent publications which describe compounds having activity as inhibitors of collagenase include the following:

US 4,235,885; US 4,297,275; US, 4,327,111 and US 4,382,081 (to Squibb); US 4,374,765; US 4,371,465; US 4,609,667; US 4,361,574 and US 4,371,466 (to American Home Products); US 4,242,354 (to Mediolanum Farm.); and WO 89/05819 (to Fuji Yakuhunin Kogyo).

We have now discovered that compounds which are inhibitors of the mammalian collagenase family of enzymes, including known inhibitor compounds and in particular the known inhibitor compounds of the formula (I) to (XIV) as hereinbefore defined, are also able to inhibit the degradation of myelin basic protein by the myelin-associated protease. These compounds and in particular compounds of the formula (I) to (XIV) as hereinbefore defined, are thus of potential utility in the treatment of demyelinating diseases such as multiple sclerosis and related demyelinating disorders.

Accordingly, the present invention provides a method of treatment of demyelinating diseases of the nervous system, in particular multiple sclerosis and related demyelinating disorders, in mammals including humans, which method comprises administering to the mammal in need of such treatment an effective amount of a compound, or a pharmaceutically acceptable salt thereof, which is an inhibitor of a member of the mammalian collagenase family of enzymes, including collagenase.

The present invention also provides a method of treatment of demyelinating diseases of the nervous system, in particular multiple sclerosis and related demyelinating disorders, in mammals including humans, which method comprises administering to the mammal in need of such treatment an effective amount of a compound of the formula (I) to (XIV) as hereinbefore defined, or a pharmaceutically acceptable salt thereof.

Pharmaceutically acceptable salts of compounds for use in the invention, including compounds of the formula (I) to (XIV), include salts formed with bases and, where a basic nitrogen is present, acid addition salts; these may be prepared by standard methods well known in the art of organic chemistry. Preferred salts for any compound identified in any of the aforementioned Patent Publications, in particular compounds of the formula (I) to XIV), are incorporated herein by reference thereto.

Compounds for use in the invention, including compounds of the formula (I) to (XIV), and their pharmaceutically acceptable salts may also form pharmaceutically acceptable solvates, including hydrates, and these are encompassed whenever a compound for use in the invention is referred to.

Compounds for use in the invention and particularly compounds of the formula (I) to (XIV) may have one or more chiral centres and are thus capable of existing in more than one stereoisomeric form. The invention extends to each stereoisomeric form and to mixtures thereof, including racemates. Preferred stereoisomeric forms, and methods for their separation, of compounds identified in any of the aforementioned Patent publications, in particular compounds of formula (I) to (XIV), are incorporated herein by reference thereto.

The above-identified compounds of the formula (I) to (XIV) are defined using standard terminology well known in the art of organic chemistry. For the avoidance of doubt and for the sake of clarity, reference may be made to the individual Patent Publications, wherein compounds of the formula (I) to (XIV) are defined, in order to determine the particular meaning to be attributed to general terms, including inter alia alkyl, alkoxy, aryl, heteroaryl, acyl, aroyl, halogen. amino acid or amino acid residue, 'optionally substituted' and

'protected', where used in the definition of compounds of the formula (I) to (XIV).

In compounds of the formula (I), preferred values for $R_a$ include hydrogen, acetyl and benzoyl, $R_c$ is preferably 2-methylpropyl and a is preferably 2, 3 or 4.

Suitable compounds of the formula (I) are those exemplified in United States Patent No. 4,263,293.

In compounds of the formula (II), preferred values for $R_d$ include methyl, phenyl and iso -propyl. Preferred values for $R_e$ include 2-methylpropyl, n -butyl and n -hexyl. $R_f$ is preferably 4-methoxybenzyl or 1-(benzyloxy)ethyl and $R_g$ is preferably methyl or n -butyl.

Suitable compounds of the formula (II) are those exemplified in United States Patent No. 4,595,700.

In compounds of the formula (III), a preferred value for $R_h$, when alkanoyl, is acetyl and, when aroyl, is benzoyl. Preferred values for $R_h$, $R_i$ and $R_j$, when alkyl, include $C_{2-6}$ alkyl with iso -butyl being particularly preferred. A preferred value for $R_j$, when aryl, is phenyl. In compounds of formula (III), $R_h$ is preferably hydrogen, $R_i$ is preferably hydrogen or methyl and $R_j$ is preferably alkyl with iso -butyl being most preferred.

The hydrophobic amino acid, $AA_1$, is suitably selected from naturally occurring amino acids such as phenylalanine, tryptophan and tyrosine, or may be a synthetic aromatic amino acid, for example naphthylalanine. Preferably $AA_2$ is alanine and $AA_3$ is glutamine. Suitable values for B include amino and ethoxy. Suitable compounds of the formula (III) are those exemplified in International Patent Application WO88/06890.

In compounds of the formula (IV), suitable values for $R_k$ include hydrogen; $C_{1-6}$ alkyl; phenyl and naphthyl optionally substituted by OH, $C_{1-6}$ alkyl, $C_{1-6}$ alkoxy and halogen; hydroxy; $C_{1-6}$ alkoxy; -NH-$CH_2$-COOH; -NH-$CH_2$-$CONH_2$; -NH-$CH_2$-$CO_2$Et; -NH-$CH_2$-$CO_2{}^tBu$; -NH-CH($^i$Bu)-$CO_2$H; -NH-CH($^i$Bu)-$CO_2{}^tBu$; and -$NR_pR_q$ in which one of $R_p$ and $R_q$ is hydrogen and the other is $C_{1-8}$ alkyl such as methyl or ethyl, or -$NR_pR_q$ is N′-methyl-N -piperazinyl or N -morpholinyl.

Preferably $R_k$ is $C_{1-4}$ alkyl, especially methyl, $C_{1-4}$ alkoxy, especially methoxy, amino, alkylamino, especially methylamino, or -NH-$CH_2$-$CO_2$H. Suitable values for $R_z$ include hydrogen, acetyl and benzoyl. Preferably $R_z$ is hydrogen.

$R_m$ is suitably a C4 alkyl group such as n-butyl, iso -butyl or sec -butyl. Preferably $R_m$ is iso -butyl. Preferred values for $R_n$ include iso -butyl, benzyl, $C_{1-6}$ alkoxybenzyl, especially 4-methoxybenzyl, 1-(benzyloxy)ethyl, and 9- or 10-membered fused bicyclic heteroarylmethyl, especially 3-indolylmethyl. Suitable values for $R_o$ include hydrogen, methyl, ethyl and 1-(methoxycarbonyl)ethyl. Preferably $R_o$ is methyl.

Suitable compounds of the formula (IV) are those exemplified in European Patent Publication Nos. 273689 and 322184. A preferred compound of formula (IV) is the compound described in Example 27 of European Patent Publication No. 273689.

In compounds of the formula (V), suitable values for $R_1$ and $R_2$ include hydrogen, $C_{1-4}$ alkyl, preferably methyl, $C_{1-4}$ alkoxy, preferably methoxy, halogen, preferably chlorine, and $CF_3$. Preferably both $R_1$ and $R_2$ are hydrogen or one of $R_1$ and $R_2$ is hydrogen and the other is $C_{1-4}$ alkyl, especially methyl, $C_{1-4}$ alkoxy, especially methoxy, halogen, especially chlorine, or $CF_3$.

Preferably, $R_3$ is hydrogen, acetyl, benzoyl or a group R-S- where R is a $C_{1-6}$ alkyl group or such that the compound of formula (V) is a dimer about the disulphide bond. More preferably $R_3$ is hydrogen.

$R_4$ is suitably a C4 alkyl group such as n -butyl, iso -butyl or sec -butyl. Preferably $R_4$ is iso -butyl.

Preferred values for $R_5$ include iso-butyl, benzyl, $C_{1-6}$ alkoxybenzyl, especially 4-methoxybenzyl, 1-(benzyloxy)ethyl, and 9- or 10-membered fused bicyclic heteroarylmethyl, especially 3-indolylmethyl. Suitable values for $R_6$ include hydrogen, methyl, ethyl and 1-(methoxycarbonyl)ethyl. Preferably $R_6$ is methyl. when $R_5$ and $R_6$ are combined as -$(CH_2)_e$-, e is preferably 10, resulting in a lactam structure based on a 13-membered ring.

Suitable compounds of the formula (V) are those exemplified in European Patent Publication No. 358305.

In compounds of the formula (VI), $R_{14}$ is preferably methyl, $R_{15}$ is preferably 4-methoxybenzyl, and D is suitably -($CHR_{16}$-$CHR_{17}$)- in which $R_{16}$ is preferably hydrogen and $R_{17}$ is preferably 2-methylpropyl.

Suitable compounds of formula (VI) are those exemplified in United States Patent No. 4,599,361 and European Patent Publication No. 214639. A preferred compound of formula (VI) is the compound described in Example 1 of United States Patent No. 4,599,361.

In compounds of the formula (VII), E is preferably L-valine and F is preferably $c_{1-2}$ alkylamino in which the alkyl moiety is substituted by phenyl or trifluoromethylphenyl.

Suitable compounds of the formula (VII) are those exemplified in European Patent Publication No. 274453.

In compounds of the formula (VIII), suitable values for $R_{18}$ include iso -butyl and n -pentyl. $R_{19}$ and $R_{20}$ are suitably independently selected and derived from the group of amino acids comprising glycine, alanine, valine, leucine, isoleucine, phenylalanine, tyrosine, tryptophan, serine, threonine, cysteine, methionine,

asparagine, glutamine, lysine, arginine, glutamic acid and aspartic acid. Suitable values for $R_{21}$ include those defined for $R_{19}$ and $R_{20}$.

Preferably, $R_{19}$ and $R_{20}$ are selected from glycine, tyrosine, tryptophan, serine, threonine, cysteine, methionine, asparagine, glutamine, lysine, arginine, glutamic aid and aspartic acid.

In preferred compounds of the formula (VIII), $R_{18}$ is iso -butyl or n -pentyl, $R_{19}$ is leucine or valine, $R_{20}$ is alanine or phenylalanine, Y is 1, and G is hydrogen. Suitable compounds of formula (VIII) are those exemplified in European Patent Publication No. 231081.

In compounds of the formula (IX), $R_a'$ is suitably a C3-or C4-alkyl group and preferably an n -propyl, iso -butyl or sec -butyl group.

$R_b'$ is suitably an iso -propyl or iso -butyl group, a protected p-hydroxybenzyl group, preferably p-methoxybenzyl, or protected or acylated 4-aminobutyl, preferably 4-tert -butoxycarbonylaminobutyl, 4-glycylaminobutyl or 4-(4-carboxybenzoylamino)butyl. $R_c'$ is suitably hydrogen, methyl or a protected 4-aminobutyl group. Preferably $R_c'$ is hydrogen, methyl or 4-phthaloylaminobutyl.

$R_d'$ is suitably hydrogen or methyl or alternatively $R_b'$ and $R_d'$ together are $-(CH_2)_f-$ where f is an integer from 5 to 9.

When $R_d'$ is hydrogen or methyl, $R_e'$ is suitably hydrogen, $C_{1-6}$ alkyl, $C_{1-6}$ alkoxy $C_{1-6}$ alkyl, di($C_{1-6}$ alkoxy)methyl, carboxy or $C_{1-6}$ alkoxycarbonyl. When $R_d'$ is hydrogen or methyl, $R_e'$ is preferably carboxy or $C_{1-6}$ alkoxycarbonyl especially ethoxycarbonyl. When $R_b'$ and $R_d'$ together are $-(CH_2)_f-$, $R_e'$ is preferably hydrogen. Preferably $R_f'$ is hydrogen.

Suitable compounds of the formula (IX) are those exemplified in European Patent Publication No. 236872.

In compounds of the formula (X) there is a preferred, group of compounds in which $R_i'$ is as hereinbefore defined but excluding aralkyl or heteroalkyl, and $K^3$ is group $-NH-CH(R_r')-C(O)R_u'$ in which $R_r'$ represents alkyl substituted by alkoxy, aralkoxy, alkoxycarbonylamino, aralkoxycarbonylamino, carboxyalkyl or carboxyaralkyl, or $R_r'$ represents aralkyl substituted by one or more groups selected from alkyl, alkoxy, alkylthio or aralkoxy, and $R_u'$ is as hereinbefore defined.

When $K^3$ is a group $-NH-OH(R_r')-C(O)R_u'$ as defined above, preferably $K^1$ and $K^2$ together represent hydrogen, L is a direct chemical bond, $R_h'$ represents hydrogen and $R_i'$ represents alkyl or alkyl substituted by one or more trifluoromethyl groups.

Within this preferred first sub-class of compounds $R_r'$ is preferably benzyloxymethyl, 1-(benzyloxy)ethyl, 4-benzyloxyphenylmethyl or 4-methoxyphenylmethyl.

In a second preferred sub-class of compounds falling within the preferred definition of $K^3$, L represents $NR_j'$ and $K^1$ and $K^2$ represent a group $R_s'CO-$ wherein $R_s'$ represents alkyl, aryl, aralkyl, aralkoxy, alkylamino, arylamino, aralkylamino, dialkylamino; or aryl, aralkyl and aralkoxy each substituted in the aromatic moiety as hereinbefore defined. Particularly preferred compounds in this second sub-class are those in which $R_j'$ is hydrogen, and $R_i'$ and $R_r'$ are as defined in the first identified sub-class of compounds.

In a most preferred group of compounds within this second sub-class, g is 2 and $R_s'$ is benzyloxy; benzyloxy substituted by 4-chloro, 2-chloro, 4-methyl, 4-nitro or 4-amino; benzylamino; phenyl; or phenyl substituted by 4-chloro, 4-chloro, 4-methyl, 4-nitro or 4-amino.

Suitable compounds of the formula (X) are those exemplified in European Patent Publication No. 126974.

In compounds of the formula (XI), $R_v'$ is suitably straight or branched chain $C_{1-6}$ alkoxy and preferably n -propoxy or n -pentoxy. $R_w'$ is suitably alkylamino and preferably methylamino.

Suitable compounds of the formula (XI) are those exemplified in European Patent Publication No. 159396.

In compounds of the formula (XII), $R_1'$ is preferably iso -butyl. Suitable values for $R_2'$ include n -butyl, n -pentyl, n -hexyl and iso -butyl. Preferably $R_2'$ is iso -butyl. Suitable values for $R_3'$ include methyl, iso - propyl, iso -butyl and phenylmethyl. Suitable values for $R_4'$ include methyl and phenylmethyl. Suitably $R_5'$ is methyl and $R_6'$ is hydrogen or methyl.

Suitable compounds of the formula (XII) are those exemplified in European Patent Publication No. 232027.

In compounds of the formula (XIII), $R_7'$ is preferably hydroxy. suitable values for $R_8'$ include hydrogen, methyl, ethyl and iso -propyl. $R_8'$ is preferably methyl or ethyl. $R_9'$ is suitably a C4 alkyl group such as n - butyl, iso -butyl or sec -butyl. Preferably $R_9'$ is iso -butyl.

Preferred values for $R_{10}'$ include benzyl, 4-hydroxybenzyl, $C_{1-6}$ alkoxybenzyl, especially 4-methoxybenzyl, and 9- or 10-membered fused bicyclic heteroaryl, especially 3-indolylmethyl. $R_{11}'$ is suitably methyl or ethyl and preferably methyl.

Suitable compounds of the formula (XIII) are those exemplified in European Patent Publication No. 320118.

A preferred compound of the formula (XIII) is the compound Example 1 A/B described in European Patent publication No. 320118.

In compounds of the formula (XIV), $R_{12}'$ is preferably hydrogen or $C_{1-6}$ alkyl, especially methyl. $R_{13}'$ is

preferably a C3- or C4-alkyl group, especially n -propyl, iso -butyl or sec -butyl. Preferably $R_{14}'$ represents iso -butyl and $R_{15}'$ represents hydrogen, or $R_{14}'$ and $R_{15}'$ together represent $-(CH_2)_n-$ where n is 5 to 9, and $R_{16}'$ represents hydrogen. Alternatively $R_{14}'$ preferably represents iso -butyl, $R_{15}'$ preferably represents methyl and $R_{16}'$ preferably represents a carboxyl or $C_{1-6}$ alkoxycarbonyl group, especially ethoxycarbonyl.

Preferred values for X are identified in European Patent Publication No. 276436.

Suitable compounds of the formula (XIV) are those exemplified in European Patent Publication No. 276436.

Compounds for use in the invention, including compounds of the formula (I) to (XIV), may be prepared as described in the aforementioned Patent Publications, the subject matter of each of which is incorporated herein by reference, or by analogous methods thereto.

The administration of compounds for use in the present invention, including compounds of the formula (I) to (XIV) and pharmaceutically acceptable salts thereof, may be by way of oral or parenteral administration, but oral administration is preferred.

Preferably a compound or a pharmaceutically acceptable salt thereof will be administered in the form of a pharmaceutical composition in which it is combined with a pharmaceutically acceptable carrier.

Preferably, a pharmaceutical composition is in unit dosage form and in a form adapted for oral or parenteral administration.

A composition of the invention, which may be prepared by admixture, may contain a diluent, binder, filler, disintegrant, flavouring agent, colouring agent, lubricant or preservative in conventional manner. These conventional excipients may be employed in conventional manner.

Compositions may, for example, be in the form of tablets, capsules, sachets, vials, powders, granules, lozenges, reconstitutable powders, or liquid preparations, for example solutions or suspensions, or suppositories.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

Solid compositions may be obtained by conventional methods of blending, filling, tableting or the like. Repeated blending operations may be used to distribute the active agent throughout those compositions employing large quantities of fillers. when the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients. For example, in a hard gelatin capsule containing the required amount of a compound of the invention in the form of a powder or granulate in intimate mixture with a lubricant, such as magnesium stearate, a filler, such as microcrystalline cellulose, and a disintegrant, such as sodium starch glycollate.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil and fractionated coconut oil, oily esters, for example esters of glycerine, propylene glycol, ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository or for parenteral administration in an injectable form. For injection, for example by injection into the cerebro-spinal fluid or via other routes that will gain access to the sites of demyelination as freely soluble solutions or as poorly dispersed depot stores, the compounds of the invention may be presented in an aqueous or non-aqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable

additives. Such forms will be presented in sterile unit dose form such as ampoules or disposable injection devices or in multi-dose forms such as a bottle from which the appropriate dose may be withdrawn or a solid form or concentrate which can be used to prepare an injectable formulation.

For topical and percutaneous administration, the preparations may also be presented as an ointment, cream, lotion, gel, spray, aerosol, wash, skin paint or patch.

The suitable dosage range for compounds for use in the invention may vary from compound to compound and may depend on the particular nature and severity of the demyelinating condition to be treated. It will also depend, inter alia , upon the relation of potency to absorbability and the mode of administration chosen.

A unit dose for demyelinating diseases will generally contain from 10 to 1000 mg and preferably will contain from 10 to 500 mg, in particular 10, 50, 100, 150, 200, 250, 300, 350, 400, 450 or 500 mg. The composition may be administered one or more times a day, for example 2, 3 or 4 times daily, so that the total daily dose for a 70 kg adult will normally be in the range 10 to 3000 mg. Such a dosage corresponds to approximately 0.15 to 50 mg/kg per day. Suitably the dosage is within the range 0.5 to 20 mg/kg per day.

Alternatively, in particular for injection, the unit dose will contain from 2 to 200 mg of a compound of the invention and be administered in multiples, if desired.

The present invention also provides the use of a compound, or a pharmaceutically acceptable salt thereof, which is an inhibitor of a member of the mammalian collagenase family of enzymes for the manufacture of a medicament for use in the treatment of demyelinating diseases of the nervous system, in particular multiple sclerosis and related demyelinating disorders.

Moreover, the present invention provides the use of a compound of the formula (I) to (XIV) as hereinbefore defined, or a pharmaceutically acceptable salt thereof, for the manufacture of a medicament for use in the treatment of demyelinating diseases of the nervous system, in particular multiple sclerosis and related demyelinating disorders.

The present invention further provides a pharmaceutical composition for use in the treatment of demyelinating diseases of the nervous system, in particular multiple sclerosis and related demyelinating disorders, which comprises an effective amount of a compound, or a pharmaceutically acceptable salt thereof, which is an inhibitor of a member of the mammalian collagenase family of enzymes and a pharmaceutically acceptable carrier.

There is also provided a pharmaceutical composition for use in the treatment of demyelinating diseases of the nervous system, in particular multiple sclerosis and related demyelinating disorders, which comprises an effective amount of a compound of the formula (I) to (XIV) as hereinbefore defined, or a pharmaceutically acceptable salt thereof, and a pharmaceutically acceptable carrier.

Such compositions may be prepared in the manner as hereinbefore described.

The following examples illustrate the preparation of representative compounds for use according to the invention of the formula (IV), (VI) and (XIII) and the following pharmacological data illustrate their activities.

## Description 1

### 3-Acetylmercapto-6-methyl-4-[[[1-(S)-[(methylamino)carbonyl ]-2-(3-indolyl)ethyl]amino]carbonyl]heptanoic acid, methyl ester (D1)

A solution of 6-methyl-4-[[[1-(S)-[(methylamino)carbonyl]-2-(3-indolyl)ethyl]amino]carbonyl]hept-2(and 3)-enoic acid, methyl ester (2.5 g; EPA 273689, Description 16) in thiolacetic acid (100 ml) was left at room temperature for 28 days, then was evaporated to dryness in vacuo . Column chromatography (150 g) of the residue eluting initially with ether, and then ether-chloroform (1:1) gave the title compound (0.32 g) as a single isomer (Isomer A), m.p. 134-136°C (from ether). (Found: C,60.32; H,6.88; N,8.80. $C_{24}H_{33}N_3O_5S$ requires C,60.61; H,6.99; N,8.83%).

$\delta$ ($CDCl_3$): 0.84(d,J=5Hz) and 0.85(d,J=5Hz) (total 6H), 1.2-1.75 (2H,m), 2.29(3H,s), 2.5-2.7(3H,m), 2.64-(3H,d,J=5Hz), 3.11(1H,dd,J=8,14Hz), 3.3(1H,dd,J=6,14Hz), 3.62(3H,s), 3.94(1H,m), 4.72(1H,g,J=7Hz), 5.64(1H,brd), 6.52(1H,d,J=8Hz) 7.05-7.25(3H,m), 7.36(1H,d,J=7Hz), 7.72(1H,d,J=7Hz) and 8.17(1H,s).

## Description 2

N-[3-(N′-Benzyloxycarboxamido)-2-(2-methylpropyl)propanoyl]-O-methyl-L-tyrosine N-Methylamide (D2)

To a solution of N-[3-carboxy-2-(2-methylpropyl)propanoyl]-O-methyl-L-tyrosine N-methylamide (US Patent 4599361; J.P. Dickens et al .) (0.106 mol) in a mixture of dimethylformamide and dichloromethane, was added 1-hydroxybenzotriazole (18g, 0.117 mol) in dimethylformamide. A solution of O-benzylhydroxylamine (18.8g, 0.117 mol) in dichloromethane, previously adjusted to pH 8.0 with diisopropylethylamine, was added and the mixture was cooled to 0°C. A solution of dicyclohexylcarbodiimide (24g, 0.117 mol) in dichloromethane was added and the mixture was stirred at room temperature for 18h.

The mixture was filtered, and the filtrate was evaporated to dryness in vacuo and dissolved in ethyl acetate. It was washed with 1 N hydrochloric acid, water, sodium bicarbonate and water and dried ($MgSO_4$). The product was chromatographed on silica gel, eluting with ethyl acetate, followed by methanol-ethyl acetate (1:19) to afford the title compound (20.3g) as a 1:1 mixture of diastereoisomers.

Example 1

3-Mercapto-6-methyl-4-[[[1-(S)-[(methylamino)carbonyl]-2-(3-indolyl)ethyl]amino]carbonyl]heptanoic acid, methyl ester (E1)

An ice-cooled solution of 3-acetylmercapto-6-methyl-4-[[[1-(S)-[(methylamino)carbonyl]-2-(3-indolyl)-ethyl]amino]carbonyl]heptanoic acid, methyl ester (01; 0.1 g) in nitrogen-purged methanol (5 ml) was treated with 35% aqueous ammonia (1.5 ml). The mixture was stirred at room temperature under nitrogen for 2 h. The solution was evaporated to dryness in vacuo and the residue was triturated with cold ether to give the title compound as a single isomer (Isomer A), (52 mg), m.p. 73-75°C. (Found: C,60.69; H,7.35; N,9.64%. $C_{22}H_{31}N_3O_4S$ requires C,60.95; H,7.21; N,9.69%).

δ ($CDCl_3$): 0.87(6H,d,J=6Hz), 1.4-1.7(2H,m), 1.67(1H,d,J=9Hz), 2.37(1H,dd,J=5,15Hz], 2.41(1H,m), 2.60-(1H,dd,J=3,15Hz), 2.65(3H,d,J=5Hz], 3.13(1H,dd,J=7,14Hz), 3.21(1H,m), 3.30(1H,dd,J=7,14Hz), 3.67-(3H,s), 4.75(1H,g,J=7Hz), 5.60(1H,brd), 6.53(1H,d,J=8Hz), 7.05-7.25(3H,m), 7.35(1H,d,J=8Hz), 7.71-(1H,d,J=8Hz) and 8.13(1H,s).

Example 2

N-[3-(N′-Hydroxycarboxamido)-2-(2-methylpropyl)propanoyl]-O-methyl-L-tyrosine N-Methylamide (E2)

A mixture of N-[3-(N′-benzyloxycarboxamido)-2-(2-methylpropyl)propanoyl]-O-methyl-L-tyrosine N-methylamide (D2) (20.3g) and 10% palladium-carbon (2g) in ethanol was hydrogenated at room temperature and atmospheric pressure until uptake of hydrogen ceased. The solution was filtered, and the filtrate evaporated to dryness in vacuo , to afford the title compound as a white solid (16g), $[\alpha]_D^{20}$ = -8.1° (c=1% in methanol).

δ ($d_6$-DMSO) 0.5-0.9 (6H, m), 1.0-1.5 (1H, m), 2.0-2.5 (2H, m), 2.5-2.8 (8H, m), 3.75 (3H, s), 4.45 (1H, m), 6.75 (2H, t, J=8Hz), 7.15 (2H, t, J=8Hz), 7.4-7.8 (2H, m), 7.85 (1H, m), 8.1 (1H, d, J=8Hz), 8.65 (1H, s) and 10.50 (1H, s).

Example 3

N-[N-(1-Phosphonoethyl)-leucyl]-N,O-dimethyl-L-tyrosinamide (E3)

N-[N-(1-Phosphonoethyl)-leucyl]-N,O-dimethyl-L-tyrosinamide, diethyl ester (0.4g; EPA 320118, Description 3) was dissolved in dichloromethane (10 ml) and treated with bromotrimethyl silane (5 ml). The solution was stirred at room temperature for 48 h, methanol (20 ml) was added and the solvent evaporated in vacuo to give the title compound, a yellow foam, as a mixture of 4 diastereoisomers (0.37 g) (E1). Column chromatography of the crude product (150 mg) using reverse phase silica, eluting with a gradient of 5% to 30% methanol in water, gave a faster running 1:1 mixture of 2 diasteroisomers. Isomer E1 A/B (30

mg).

δ ($CDCl_3$): 0.80(6H,m), 1.16(4H,m), 1.44(3H,m), 2.58(3H,m), 2.78(1H,m), 2.98(1H,m), 3.52(0.5H, broad s), 3.71(3H, s), 3.88(0.5H, broad s), 4.46(1H, m), 6.82(2H, m), 7.16(2H,m), 8.18(1H, broad s), 8.80(1H, m). Observed FAB $(M+H)^+$ 430. $C_{19}H_{32}N_3O_6P$ requires M 429.

Further elution gave a slower running mixture of 2 diastereoisomers.

Isomer E1 C/D (20 mg). Observed FAB $(M+H)^+$ 430. $C_{19}H_{32}N_3O_6P$ requires M 429.

Separation of Isomers E1 A/B

Isomer E1 A/B was separated into single diastereoisomers by preparative HPLC on a Hamilton PRP-1 column, eluting with 25:75 methanol/0.05 M aqueous ammonium acetate, pH 5.0, to give:

Isomer A: δ ($CD_3OD$): 0.87(3H,d), 0.92(3H,d), 1.26(3H,dd), 1.45(3H,m), 2.65(1H,dd), 2.70(3H,s), 2.84-(1H,dd), 3.10(1H,dd), 3.63(1H, broad t), 3.74(3H,s), 4.60(1H,dd), 6.82(2H,d), 7.16(2H,d).

Isomer B: δ ($CD_3OD$): 0.90(3H,d), 0.94(3H,d), 1.25(3H,dd), 1.54(3H,m), 2.61(1H,dd), 2.69(3H,s), 2.87-(1H,dd), 3.05(1H,dd), 3.75(3H,s), 4.16(1H, broad t), 4.58(1H,dd), 6.84(2H,d), 7.18(2H,d).

Assay for inhibition of myelin-degrading metalloprotease:

Myelin-degrading enzymic activity was monitored by determining the degradation of purified, radiolabelled human MBP (myelin basic protein). Human MBP was isolated from human myelin according to the method of Dunkley and Carnegie (Research Methods in Neurochemistry, 2 , 219-245, 1974), and labelled with $^{125}$I-iodine: free iodide was removed by chromatography on Sephadex G25.

25 µl $^{125}$I-MBP (18.75 µg, $10^5$ cpm) was added to 50 µl of a solution of purified myelin-degrading enzyme (final concentration 10 µg/ml) in 150 mM Tris-HCl buffer, pH 7.4, containing 2 mM $CaCl_2$ and 2 mM $MgCl_2$. Inhibitors were dissolved in methanol and diluted in the Tris/$CaCl_2$/$MgCl_2$ buffer immediately before use.

After 2h incubations at 37°C, enzymic action was terminated by adding 25 µl SDS-sample buffer (10% SDS, 10% sucrose, 200 mM dithiothreitol and 0.02% bromophenol blue) and heating to 70°C for 10 min. An aliquot of the resulting solution was then electrophoresed on 15% polyacrylamide gels, in the presence of SDS, and the gels were stained with Coomassie Blue. Bands corresponding to intact MBP and to the large fragments generated by enzymic degradation, as described by Glynn et al. (J. Neurochem. 48 , 752-759, 1987), were excised and counted in a gamma counter.

$IC_{50}$ data were calculated as the concentrations of inhibitors reducing the activity of the myelin-degrading enzyme by 50%.

The activities of representative compounds of the invention are illustrated in the Table below:

| Inhibition of myelin-degrading enzyme | |
|---|---|
| Compound | $IC_{50}$ (µM) |
| TIMP | 90 units/ml* |
| Example 1 | 1.8 |
| Example 2 | 2.0 |
| Example 3 | 15.5 |

*The TIMP unit is defined as the amount of purified TIMP that will inhibit the enzymic activity of 2 units of mammalian collagenase by 50%. The unit of collagenase is defined as the amount of enzyme that degrades 1 µg of native fi rillar collagen (rat skin type I) per minute at 37°C.

**Claims**

1. The use of a compound, or a pharmaceutically acceptable salt thereof, which is an inhibitor of a member of the mammalian collagenase family of enzymes for the manufacture of a medicament for use in the treatment of demyelinating diseases of the nervous system.

2. Use as claimed in claim 1 wherein the demyelinating disease is multiple sclerosis.

3. Use as claimed in claim 1 or 2 wherein the compound is a collagenase inhibitor.

4. Use as claimed in claim 1, 2 or 3 wherein the compound is of the formula (I) to (XIV) as described in: US 4,263,293; US 4,595,700; WO 88/06890; EPA-273689; EPA-322184; EPA-358305; EPA-214639; EPA-274453; EPA-231081; EPA-236872; EPA-126974; EPA-159396; EPA-232027; EPA-320118 or EPA-276436.

5. Use as claimed in claim 1, 2 or 3 wherein the compound is:
3-mercapto-6-methyl-4-[[[1-(S)-[(methylamino)carbonyl]-2-(3-indolyl)ethyl]amino]carbonyl]heptanoic acid, methyl ester;
N-3-(N′-hydroxycarboxamido)-2-(2-methylpropyl)propanoyl]-O-methyl-L-tyrosine N-methylamide; or
N-[N-(1-phosphonoethyl)-leucyl]-N,O-dimethyl-L-tyrosinamide.